# EUROPEAN PATENT APPLICATION

(11) **EP 2 574 318 A1**
(43) Date of publication of application: **03.04.2013**
(21) Application number: 12186271.8
(22) Date of filing: 27.09.2012
(51) Int. Cl.: A61F 9/00, A61F 9/008

(54) **Ophthalmic laser surgical apparatus**

(30) Priority: 30.09.2011 JP 2011218701
(71) Applicant: NIDEK CO., LTD, Gamagori-shi Aichi 443-0038 (JP)
(72) Inventor: Iwata, Shinya, Gamagori-shi, Aichi, 443-0035 (JP); Murakami, Naho, Toyokawa-shi, Aichi, 441-0203 (JP)
(74) Representative: Zimmermann, Tankred Klaus

(57) **Abstract**

An ophthalmic laser surgical apparatus includes a laser source and an irradiation optical system with a beam expander unit. The beam expander unit includes a first lens unit, a second lens unit, and a plane-to-plane distance changing unit. The first lens unit is configured to change the focal length so as to change a spot size of a laser in a target position. The second lens unit includes at least one lens. The plane-to-plane distance changing unit is configured to change the distance between a principal plane of the first lens unit and a principal plane of the second lens unit so as to adjust one of divergence and convergence of the laser that has passed through the first lens unit.

## Description

### BACKGROUND

### 1. Technical Field

The present disclosure relates to an ophthalmic laser surgical apparatus that irradiates a patient's eye with a laser to remove a tissue, for example, by cutting the tissue. The present disclosure also relates to an optical system used in the ophthalmic laser surgical apparatus.

### 2. Related Art

Nowadays, a proposed technique performs irradiation of an ultrashort pulse laser beam such as a femtosecond pulse laser beam to cut (break up) a tissue of a crystalline lens or the like of an eye of a patient (patient's eye) (see JP-T-2010-538699, for example). The apparatus disclosed in JP-T-2010-538699 generates micro plasma in a target position (position of a laser spot) of a crystalline lens to mechanically cut and break up a crystalline lens tissue, so as to treat a cataract. The apparatus removes the tissue and then inserts an intraocular lens and a similar object into the eye, thus treating the cataract. A known apparatus irradiates a cornea of a patient's eye with a laser to cut a cornea tissue (see JP-T-2011-507559, for example). The apparatus disclosed in JP-T-2011-507559 performs irradiation of the aforementioned pulse laser to cut a cornea tissue to form a lens shape corresponding to an amount of refractive correction. The refractive correction is performed such that the lens-shaped tissue is taken out through incision at the cornea to change a shape of a cornea surface.

Accordingly, in the case where the ultrashort pulse laser is used to make an incision in or break up the cornea or the crystalline lens, it is preferred to employ efficient laser irradiation depending on its object. For example, cataract treatment may employ laser irradiation with comparatively large spot diameter so as to quickly treat the crystalline lens. In contrast, in the case where the laser irradiation is performed to correct refractive power of the cornea, higher irradiation accuracy is preferred (incision accuracy using a laser). Accordingly, it is preferred that the spot size be relatively small. From the view point of efficiency and accuracy of treatment, even in the case where the pulse laser is applied to break up the crystalline lens, the laser irradiation may be performed such that the spot diameter around a crystalline lens capsule is different from the spot diameter around the center of a crystalline lens nucleus.

A technical problem of the present disclosure is to provide an ophthalmic laser surgical apparatus that allows to easily change the spot size of a laser spot so as to perform an efficient surgery, and to provide an optical system of the ophthalmic laser surgical apparatus.

### SUMMARY

The present disclosure has the following configuration to solve the aforementioned problem. An ophthalmic laser surgical apparatus is for at least one of cutting and breaking up (fragmenting) a tissue of a patient's eye using a laser. The ophthalmic laser surgical apparatus includes a laser source and an irradiation optical system. The laser source is configured to emit a pulse laser that achieves a breakdown (optical breakdown) in a spot where the pulse laser is condensed. The irradiation optical system is configured to irradiate a target position with a laser. The irradiation optical system includes a moving optical system. The moving optical system three-dimensionally moves a spot of the laser. The irradiation optical system includes an objective lens that forms the spot of the laser in the target position. The moving optical system includes an optical scanner and a beam expander unit. The optical scanner is disposed in an upper stream of the objective lens. The optical scanner is configured to scan the spot of the laser on an XY plane perpendicular to an optical axis. The beam expander unit is disposed in an upper stream of the optical scanner. The beam expander unit is configured to change the beam diameter of the laser, and change one of divergence and convergence. The beam expander unit includes a first lens unit, a second lens unit, and a plane-to-plane distance changing unit. The first lens unit is configured to change the focal length so as to change the spot size of the laser in the target position. The second lens unit includes at least one lens. The plane-to-plane distance changing unit is configured to change a distance between a principal plane of the first lens unit and a principal plane of the second lens unit so as to adjust one of divergence and convergence of a laser that has passed through the first lens unit.

The present disclosure allows to easily change the spot size of the laser spot to perform an efficient surgery.

### BRIEF DESCRIPTION OF THE DRAWINGS

FIG. 1 is a configuration diagram illustrating an ophthalmic laser surgical apparatus according to an embodiment;
FIG. 2 is a configuration diagram illustrating a beam expander unit.
FIG. 3A is a diagram illustrating a change in spot size of a laser spot;
FIG. 3B is a diagram illustrating a change in spot size of the laser spot;
FIG. 4A is a diagram illustrating a change in position of the laser spot in the Z direction;
FIG. 4B is a diagram illustrating a change in position of the laser spot in the Z direction; and
FIG. 5 is a diagram illustrating a laser irradiation area of a crystalline lens.

### DETAILED DESCRIPTION

In the following detailed description, for purpose of explanation, numerous specific details are set forth in order to provide a thorough understanding of the disclosed embodiments. It will be apparent, however, that one or more embodiments may be practiced without these specific details. In other instances, well-known structures and devices are schematically shown in order to simplify the drawing.

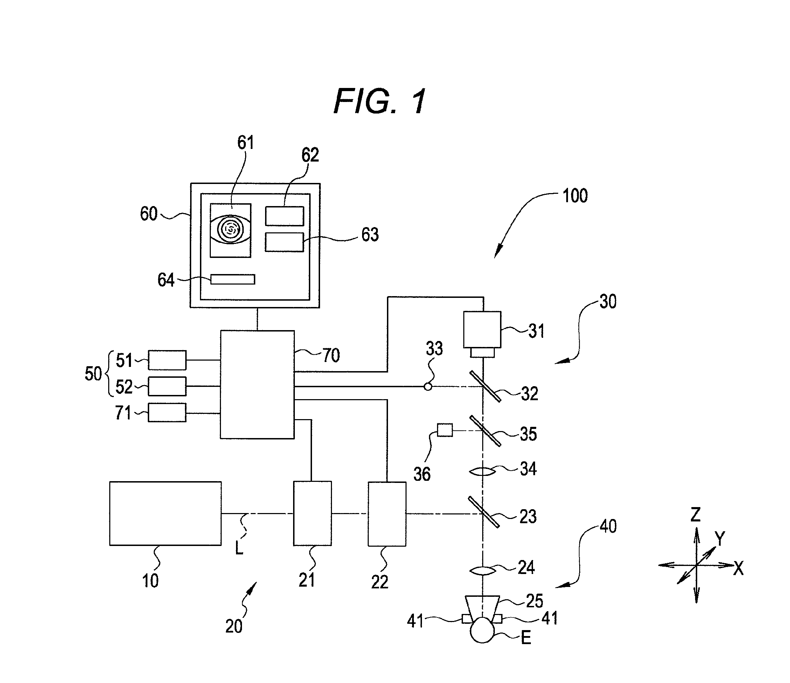

Hereinafter, an embodiment according to the present disclosure will be described with reference to the accompanying drawings. FIG. 1 is a schematic configuration diagram illustrating an ophthalmic laser surgical apparatus according to the embodiment of the present disclosure. The ophthalmic laser surgical apparatus according to this embodiment cuts and breaks up a crystalline lens of a patient's eye with a laser.

An ophthalmic laser surgical apparatus 100 includes a laser unit (laser source) 10, a laser irradiation optical system (laser irradiation unit) 20, an observing optical system (observing unit) 30, an eyeball fixing unit 40, an operating unit 50, a monitor 60, and a controller (control means) 70. The laser unit (laser source) 10 emits a pulse laser that has a characteristic to achieve a breakdown at a focal point. The laser irradiation optical system 20 guides a laser so as to irradiate a target (eyeball tissue) with the laser. The observing optical system 30 is used to observe a patient's eye. The eyeball fixing unit 40 fixes and holds the patient's eye. The operating unit 50 operates the apparatus 100. The monitor 60 is display means that performs setting and verification of the apparatus 100 and a similar process. The controller 70 integrally controls the apparatus 100.

The laser unit 10 is a laser source that emits an ultrashort pulse laser that generates plasma (achieves a breakdown) at a focal point of the laser (laser spot). As the laser unit 10, a device that emits a pulse laser with the pulse width of several femtoseconds to several picoseconds is used. At a focal point of the pulse laser, plasma is generated so as to make an incision in or break up (fragment) a target tissue. Here, an eyeball tissue such as a crystalline lens and a cornea is assumed to be the target tissue. As the laser unit 10 of this embodiment, a laser source that emits a laser is used. The laser has output power that achieves a breakdown in the case where the spot size of the laser spot is 1 to 15 µm. The laser has the pulse width of around 1 to 1000 fs (femtoseconds). It is preferred that the pulse width be equal to or less than 500 fs.

The irradiation optical system 20 includes a beam expander unit 21 (hereinafter referred to as simply expander), an optical scanner (scanning means) 22, a beam combiner 23, an objective lens 24, and an applicator 25. The expander 21 is focal point moving means that moves the laser spot of the pulse laser along the depth direction (the Z direction and the direction of the optical axis L of the laser) of the patient's eye E. The optical scanner 22 scans (moves) the laser spot on a target surface (surface perpendicular to the optical axis) two-dimensionally (in the XY direction). The beam combiner 23 aligns the optical axis of the laser with an observation optical axis. The objective lens 24 is an imaging optical system that forms an image on the target surface with a laser (forms the laser spot on the target surface). The applicator 25 is brought in contact with the patient's eye E. The expander 21 and the optical scanner 22 constitute the moving optical system that three-dimensionally moves the laser spot in the crystalline lens of the patient's eye E. Arranging the expander 21 in the upper stream reduces the swing angle of the optical scanner 22, thus reducing the effective diameter of an optical element in the downstream of the optical scanner 22. This avoids increase in size of the apparatus.

The expander 21 is disposed in the upper stream (at the laser unit 10 side) of the optical scanner 22. The expander 21 has the following functions. One function moves a plurality of lens groups (or a part of them), which are optical elements, along the optical axis so as to move (change its position) the laser spot along the Z direction. Another function changes the spot size of the laser spot, and also changes the incidence angle and the focal depth of the laser with respect to the target surface. The expander 21 changes the beam diameter of the laser guided to the optical scanner 22 and the objective lens 24, and also changes the divergence angle or the convergence angle (divergence or convergence) of the laser beam (details will be described later). As the optical scanner 22, two galvanometer mirrors where their rotation axes are mutually orthogonal are used. This allows to scan the laser spot on a predetermined two-dimensional plane (XY plane). As the optical scanner 22, a resonant mirror, a rotating prism, a scanner that is a combination of a polygon mirror and a galvanometer mirror, and a similar member may be used. The beam combiner 23 is a dichroic mirror that has the following characteristic. The dichroic mirror reflects a laser, and transmits illuminating light (including a projected target image) and reflected light of the illuminating light. The objective lens 24 forms an image of the pulse laser on the target surface using the micro spot diameter of several µm to several tens of µm. The applicator 25 includes a cup that covers a peripheral area of an anterior segment. The cup is filled with liquid so as to reduce the refractive power of the cornea. Accordingly, the applicator 25 serves as a contact lens.

The breakdown in the laser spot position causes a mechanical destruction (such as cracking) with the size of nearly the spot size in the eyeball tissue. The laser spot is moved in the Z direction by the expander 21, and moved in the XY direction by the optical scanner 22, thus being three-dimensionally moved (its position is changed). In the eyeball tissue, the laser spot is three-dimensionally moved so that the respective spots are coupled. This cuts the eyeball tissue to form a three-dimensional shape (preliminarily set pattern of laser irradiation).

The observing optical system 30 includes a camera 31 with a two-dimensional imaging device, a beam combiner 32, an illuminating light source 33, and an optical element 34. The optical element 34 is a light guiding optical system that guides the illuminating light and the light reflected at the patient's eye E. The beam combiner 32 has a characteristic that reflects the illuminating light and transmits the reflected light from the patient's eye E. Here, the beam combiner 32 is a half mirror. The illuminating light source 33 emits illuminating light such as visible light that is suitable for illuminating the patient's eye. The observing optical system 30 shares the objective lens 24. A target projecting unit 36 projects light that corresponds to the observation optical axis by a beam combiner 35. The target projecting unit 36 projects a target image on the anterior segment of the patient's eye E so as to align (position) the apparatus 100 (irradiation optical system 20). Adjustment (alignment and focus adjustment) of the apparatus 100 is performed by driving means (not illustrated) such that the laser irradiation optical system 20 and the observing optical system 30 are moved upward and downward. The two-dimensional imaging device of the camera 31 is, for example, an imager that has sensitivity to wavelengths of the illuminating light and the target image to be projected. The illuminating light source 33 may be a light source that emits infrared light.

The eyeball fixing unit 40 includes an absorption ring 41 and an applicator 25. The absorption ring 41 is a circular material, and has a shape in contact with a sclera of the anterior segment. The absorption ring 41 is sucked by a pump (not illustrated) or a similar member. This fits the eyeball to the absorption ring 41, thus fixing and holding the patient's eye E. The applicator 25 is shared by the eyeball fixing unit 40.

The operating unit 50 includes input means 51 and a foot switch 52. The input means 51 is used for setting of the apparatus 100. The foot switch 52 is used for inputting a trigger signal of the laser irradiation. The input means 51 is a computer mouse, a keyboard, and a similar device. The computer mouse is a pointing device that sets conditions such as a surgery condition on a setting screen displayed on the monitor 60. The keyboard allows to input a numerical value and information such as the surgery condition.

The monitor 60 includes a frontal image displaying portion 61, a spot size setting portion 62, a surgery mode setting portion 63, a surgery condition setting portion 64, and a similar portion. The frontal image displaying portion 61 displays a frontal image of the patient's eye taken by the camera 31. The spot size setting portion 62 displays the spot size of the laser spot, and inputs a signal to change the spot size. The surgery mode setting portion 63 displays whether a surgery mode of the apparatus is a cornea surgery mode or a crystalline lens surgery mode, and inputs a signal to select the respective surgery modes. The surgery condition setting portion 64 sets a surgery condition including a laser irradiation pattern. The spot size setting portion 62 in this embodiment selects any one of predetermined spot sizes of 5 µm, 10 µm, and 15 µm, thus inputting a signal to change the spot size.

The controller 70 includes a CPU (Central Processing Unit), and is coupled to the laser unit 10, the expander 21, the optical scanner 22, the camera 31, the illuminating light source 33, and the target projecting unit 36. The controller 70 is coupled to a memory 71 that stores, for example, a control program, a pattern of laser irradiation, a set surgery condition, and a taken image. The memory 71 stores measurement data obtained by another measurement device and similar data. The memory 71 stores a laser emission condition (controlling condition of laser emission corresponding to the spot size) of the laser unit 10. The controller 70 detects the spot size based on a signal from the spot size setting portion 62. The controller 70 controls the laser unit 10 corresponding to the spot size based on the set spot size and the laser emission condition. Specifically, in the case where the spot size is the predetermined small size, the laser unit 10 is controlled to reduce the output power (increase a threshold value of the breakdown). This control increases the repetition frequency of the laser unit 10 (for example, laser emissions per second). In contrast, in the case where the spot size is the predetermined large size, the laser unit 10 is controlled to increase the output power (decrease the threshold value of the breakdown). This control reduces the repetition frequency of the laser unit 10. These controls are performed to adjust energy of the laser that concentrates in the spot size so as to appropriately perform surgery. In the case where the spot size is small, the energy concentrates within the laser spot. Therefore, the laser output power may be low. In contrast, in the case where the spot size is large, there is a need for increasing the energy within the laser spot. Additionally, in the case where the spot size is small, scanning in the XY direction tends to cause a larger spacing between the laser spots. There is a need for having the small distance between the laser spots by increasing the repetition frequency of the laser emission. Accordingly, even in the case where the repetition frequency of the laser emission is changed, moving speed (scanning speed) of the laser spots of the expander 21 and the optical scanner 22 does not need to be changed. In this embodiment, the set spot size is assumed to be small when it is 5 µm, and assumed to be large when it is 10 or 15µm.

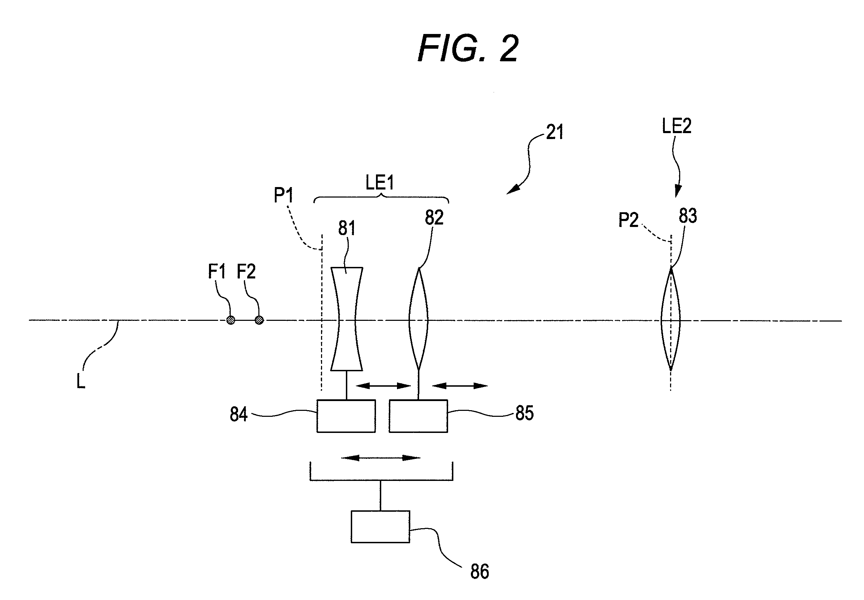

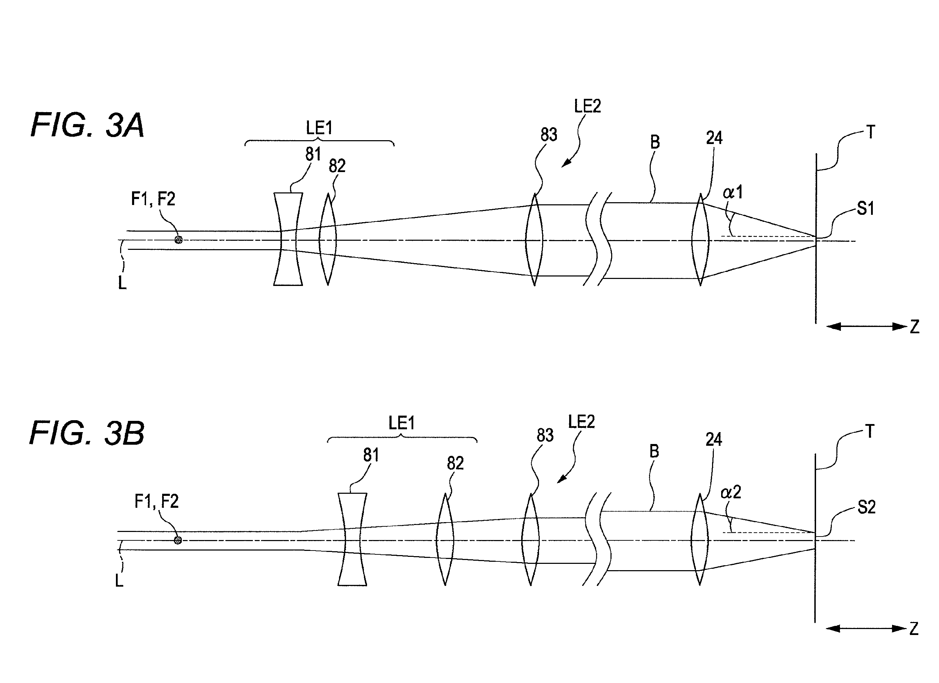

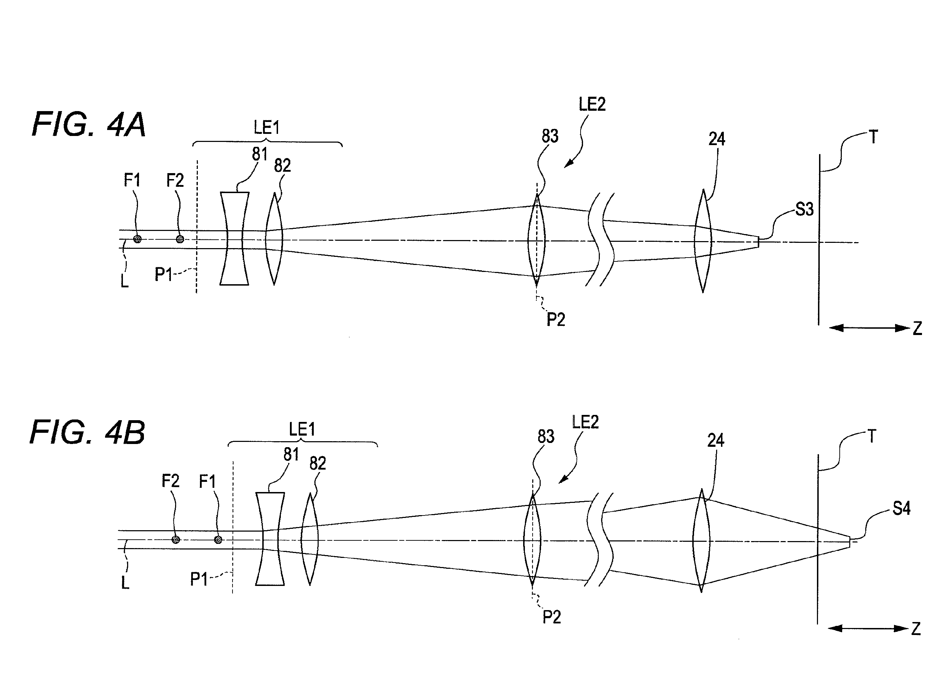

Next, a configuration and an operation of the expander (beam expander unit) 21 will be described. FIG. 2 is a configuration diagram illustrating the expander 21. FIGS. 3A and 3B are diagrams illustrating changes in spot size of the laser spot. FIGS. 4A and 4B are diagrams illustrating changes in position of the laser spot in the Z direction. In the following description, a single lens is also described as a lens group. In FIGS. 3A, 3B, 4A, and 4B, for ease of explanation, a part of the optical system such as the optical scanner 22 is omitted.

The expander 21 roughly includes two lens groups (a first lens group and a second lens group). In this embodiment, the first lens group (a first lens unit) and the second lens group (a second lens unit) are arranged from upper stream to downstream in this order. The first lens group has a configuration that allows to change the focal length. The expander 21 in this embodiment includes focal length changing means and plane-to-plane distance changing means (unit). The focal length changing means changes the focal length of the first lens group. The plane-to-plane distance changing means changes the distance between a principal plane of the first lens group and a principal plane of the second lens group. By changing the focal length of the first lens group with the focal point at an image side of the first lens group corresponding to the focal point at an object side of the second lens group, the beam diameter of the laser emitted from the expander 21 is changed. This changes the spot size of the laser spot. Changing the distance between the principal plane of the first lens group and the principal plane of the second lens group allows to change the beam divergence angle or the beam convergence angle (divergence or convergence) of the laser emitted from the expander 21. In other words, the focal length of the first lens group and the focal length of the second lens group are not changed while the distance between the first lens group and the second lens group is changed. This changes the position of the laser spot in the Z direction. Therefore, the first lens group has a function that changes the beam diameter of the laser. The second lens group has a function that adjusts convergence or divergence of the beam after the first lens group.

The expander 21 includes three lenses arranged from upper stream to downstream. The lens groups are a concave lens 81, which diffuse a beam, a convex lens 82, which condenses the beam, and a convex lens 83, which also condenses the beam. The expander 21 includes zoom units 84 to 86. The zoom unit 84 moves the concave lens 81 along an optical axis L. The zoom unit 85 moves the convex lens 82 along the optical axis L. The zoom unit 86 moves the zoom units 84 and 85 and the lenses 81 and 82 along the optical axis L. The zoom units 84 to 86 each include driving means such as a stepping motor that moves the lens and the zoom unit in accordance with a command from the controller 70.

The concave lens 81 and the convex lens 82 have focal lengths different from each other. The lenses 81 and 82 constitute a lens group (first lens group LE1) that has a negative refractive power and changes the distance between the concave lens 81 and the convex lens 82 so as to change the focal length of the first lens group LE1. The first lens group LE1 includes the zoom units 84 and 85, thus constituting the first lens unit. The first lens group LE1 has a function that changes a magnification rate of the laser beam entering from the laser unit 10. The convex lens 83 constitutes a lens group of a second lens group LE2 (second lens unit) that has a positive refractive power. The first lens group LE1 has a focal point illustrated as a focal point F1 at an image side while the second lens group LE2 has a focal point illustrated as a focal point F2 at an object side. The position of the focal point F1 (position of the first lens group LE1 with respect to the principal plane) is changed by the zoom units 84 and 85 (the focal length of the first lens group LE1 is changed). Specifically, the zoom units 84 and 85 change a relative positional relationship (distance) between the concave lens 81 and the convex lens 82. This changes the focal length of the first lens group LE1 (distance between the principal plane and the focal point F1). The second lens group LE2 is fixedly disposed, and has the constant focal length. Accordingly, the position of the focal point F2 is not changed. The zoom units 84 and 85 are arranged on the zoom unit 86 together with the lenses 81 and 82. In view of this, driving of the zoom unit 86 moves the first lens group LE1 relatively with respect to the second lens group LE2, thus changing their positional relationship. Therefore, the distance between a principal plane P1 of the first lens group LE1 and a principal plane P2 of the second lens group is changed. While the focal length of the first lens group LE1 is not changed, the relative positional relationship between the focal point F1 and the focal point F2 is changed. The zoom units 84 and 85 constitute first focal length changing means while the zoom unit 86 constitutes second focal position changing means that changes the position of the focal point F1 with respect to the focal point F2.

Next, a change in spot size of the laser spot will be described. As illustrated in FIGS. 3A and 3B, while the focal point F1 corresponds to the focal point F2, the focal length of the first lens group LE1 (the lenses 81 and 82) is changed. The zoom units 84, 85, and 86 change positions of the lenses 81 and 82 along the optical axis L. The following description will describe examples where laser spots (S1 and S2) are formed (an image is formed) on a target surface T.

FIG. 3A illustrates an example where the spot size of the laser spot S1 is small. The focal length of the first lens group LE1 is relatively shorter than that of FIG. 3B. In contrast, the distance between the convex lens 82 and the convex lens 83 are relatively longer. In view of this, a beam B enters the concave lens 81 as parallel light, and its diameter is enlarged until the beam B reaches the convex lens 83. The beam B is assumed to be a laser emitted from the focal point F2 (focal point F1), thus being collimated to parallel light by the convex lens 83. The objective lens 24 forms an image of the beam B on the target surface T. At this time, the spot size of the laser spot is assumed to be the laser spot S1. The objective lens 24 condenses (forms the image) the beam B with the wide beam diameter on the target surface T. This enlarges the incidence angle (NA) α1 (incidence angle on the target surface with respect to the optical axis) of the beam to the target surface T (the incidence angle on the target surface T with respect to the optical axis becomes large). The focal depth of the laser spot S1 becomes shallow.

FIG. 3B illustrates an example where the spot size of the laser spot S2 is large. In FIG. 3B, the focal length of the first lens group LE1 is relatively longer than that of FIG. 3A. In contrast, in FIG. 3B, the distance between the convex lens 82 and the convex lens 83 is relatively shorter than that of FIG. 3A. In view of this, the beam B enters the concave lens 81 as parallel light, and its diameter is not relatively enlarged until the beam B reaches the convex lens 83. The beam B is assumed to be a laser emitted from the focal point F2 (focal point F1), thus being collimated to parallel light by the convex lens 83. The objective lens 24 forms an image of the beam B on the target surface T. At this time, the spot size of the laser spot is assumed to be the laser spot S2. The objective lens 24 condenses (forms the image) the beam B with the narrow beam diameter on the target surface T. This reduces the incidence angle (NA) α2 with respect to the target surface T (incidence angle of the beam on the target surface T with respect to the optical axis). The focal depth of the laser spot S2 becomes deep.

The objective lens 24 receives the beams B that has the same divergence angle (here, the divergence angle of the parallel light) of the beam and the different beam diameter. The objective lens 24 has the constant focal length. Accordingly, images of the beams B are formed in a position (here, on the target surface T) with the constant distance from the objective lens 24.

While this changes the focal length of the first lens group LE1, the focal point F1 of the first lens group is made to correspond to the focal point F2 of the second lens group. This consequently changes the spot size of the laser spot and the incidence angle (NA) with respect to the target surface without changing the position of the laser spot in the Z direction. This also changes the focal depth of the laser spot.

Next, a change in position of the laser spot in the Z direction will be described. As illustrated in FIGS. 4A and 4B, the relative positional relationship between the principal plane P1 of the first lens group LE1 and the principal plane P2 of the second lens group LE2 is changed. This changes the beam divergence angle or the beam convergence angle of the laser emitted from the expander 21. The following description will describe an example where an image of a laser spot S3 is formed at the back side (rear side) of the target surface T, and another example where an image of a spot S4 of the laser spot is formed at the front side of the target surface T. Here, the first lens group LE1 is assumed to have the constant focal length. That is, the distance between the concave lens 81 and the convex lens 82 is assumed to be constant. In this state, the first lens group LE1 is moved along the optical axis L. Since the first lens group LE1 has the constant focal length, the spot sizes are the same in FIGS. 4A and 4B.

In FIG. 4A, the principal plane P1 and the principal plane P2 are relatively far from each other. In other words, the focal point F1 is located in the upper stream of the focal point F2. The distance between the principal plane P1 and the principal plane P2 is longer than the sum of the focal length of the first lens group LE1 and the focal length of the second lens group LE2. As illustrated in the drawings, the position of the first lens group LE1 is changed. In the case where the distance between the principal plane P1 and the principal plane P2 corresponds to the sum of the focal length of the first lens group LE1 and the focal length of the second lens group LE2, an image of the laser spot is formed on the target surface T (in the state of FIGS. 3A and 3B). The beam B emitted from the convex lens 83 side with respect to the focal point F2 reaches the convex lens 83. Therefore, the beam B is emitted as converging light from the convex lens 83. The objective lens 24 forms an image of the beam B, which is the converging light, at the front side (upper stream side) with respect to the target surface T (in an image forming position (see FIGS. 3A and 3B) in the case where the parallel light enters the objective lens 24) as the laser spot S3.

On the other hand, in FIG. 4B, the distance between the principal plane P1 and the principal plane P2 is short. In other words, the focal point F1 is located in the downstream of the focal point F2. As illustrated in the drawings, the position of the first lens group LE1 is changed. The beam B emitted from the laser unit 10 is refracted by the refractive power of the first lens group LE1 and then reaches the convex lens 83. The beam B is emitted as a diverging light from the convex lens 83. The objective lens 24 forms an image of the beam B, which is the diverging light, at the back side (rear side) with respect to the target surface T as the laser spot S4.

In view of this, the distance between the principal plane P1 and the principal plane P2 is changed. This changes the position of the laser spot in the Z direction without changing the spot size of the laser spot.

While in the description of FIGS. 3A, 3B, 4A, and 4B, the spot size of the laser spot and the position in the Z direction are independently changed, the spot size of the laser spot and the position in the Z direction may be concurrently changed.

An operation of an apparatus with the aforementioned configuration will be described. Here, an example of a crystalline lens surgery will be described. Before the surgery, an operator sets a condition and a pattern of the surgery. The operator operates the surgery mode setting portion 63 so as to set a surgery mode as a crystalline lens surgery mode. The controller 70 determines an upper limit and a lower limit in a moving range of the laser spot in the Z direction based on a signal from the surgery mode setting portion 63. Subsequently, the controller 70 determines the moving range (scanning range) of the laser spot in the Z direction in the surgery of the patient's eye E based on measurement data of the patient's eye, which is preliminarily stored in the memory 71. The measurement data of the patient's eye may be acceptable if it is measurement data to acquire information of the patient's eye in the depth direction, and for example, if it includes positions (relative distance from a cornea's anterior surface) of an anterior capsule and a posterior capsule of the crystalline lens. The positions of the anterior capsule and the posterior capsule of the crystalline lens are obtained from the measurement data from an ocular axial length measurement device. The measurement data may be similarly obtained from an image obtained by an optical coherence tomography device or measurement data obtained by ultrasonic diagnostic equipment and similar equipment. The moving range of the laser spot in the Z direction is assumed to be the distance between a position ahead of the posterior capsule of the crystalline lens in a predetermined range (for example, 500 µm) and the anterior capsule.

Subsequently, the operator operates the spot size setting portion 62 so as to determine the spot size. The controller 70 determines the spot size of the laser spot to be applied in the surgery based on the signal for changing (setting) the spot size. In the crystalline lens surgery, high process accuracy is not needed. Since the object is breaking up the crystalline lens nucleus, it is preferred that laser irradiation be performed with large spot size, which is set to 15 µm here.

The controller 70 controls the zoom units 84, 85, and 86 based on the moving range of the laser spot in the Z direction and the spot size of the laser spot. Specifically, the controller 70 controls the zoom units 84 and 85 to set the spot size to 15 µm, and then determines the focal length of the first lens group LE1. The controller 70 also controls the zoom unit 86 to determine the moving range of the first lens group LE1.

In addition, the controller 70 controls the laser unit 10 corresponding to the set spot size (sets the laser emission condition). Here, the controller 70 controls the laser unit 10 so as to increase laser output power corresponding to large spot size, and reduce repetition frequency.

The operator operates the surgery condition setting portion 64 so as to set the irradiation pattern and a similar parameter. The operator lays the patient on an operating table and the like, and fixes the eyeball with the eyeball fixing unit 40. The patient's eye is held by the absorption ring 41. The operator fills the applicator 25 with liquid. Subsequently, the operator operates a switch (not illustrated) so as to project a target on the patient's eye E from the target projecting unit 36. The operator performs alignment of the apparatus 100 while confirming the target and the anterior segment image of the patient's eye E displayed on the frontal image displaying portion 61.

When the foot switch 52 is pushed by the operator, the controller 70 starts laser irradiation based on a trigger signal. The controller 70 applies the laser based on the set condition and pattern of the surgery. The controller 70 controls the laser unit 10 based on the pattern, and also controls the expander 21 (zoom unit 86) and the optical scanner 22. At this time, the controller 70 moves the laser spot from the back side to the front side in the pattern. The crystalline lens of the patient's eye E is broken up. When the laser irradiation is terminated, the broken-up crystalline lens is removed by another device of an irrigation and aspiration device. An intraocular lens is installed in a capsule of the patient's eye E. Then, the surgery ends.

While the crystalline lens of the patient's eye is broken up in the above description of the embodiment, the embodiment is not limited to this. Any other configuration is possible insofar as the eyeball of the patient's eye is irradiated with the laser so as to make an incision in the tissue, and break up the tissue. The cornea surgery mode may be set through the surgery mode setting portion 63. In this case, the controller 70 determines the upper and lower limits of the moving range of the laser spot in the Z direction based on the signal for setting the surgery mode. The cornea surgery needs fine processing. Accordingly, it is preferred that the spot size of the laser spot be small.

The simple configuration described above allows to change the spot size of the laser spot, thus performing an efficient surgery. Specifically, in the crystalline lens surgery which does not require high process accuracy, this configuration increases the spot size so as to break up the crystalline lens, thus reducing surgery time. In this case, the focal depth of the laser spot becomes deeper corresponding to the enlarged spot size. This allows to efficiently break up the crystalline lens nucleus in the depth direction. In the cornea surgery which requires high process accuracy, this configuration reduces the spot size so as to make an incision in (excise) the cornea. In this case, the focal depth of the laser spot becomes shallower corresponding to reduction in the spot size. This reduces the amount of incision in the depth direction and performs a process with high accuracy. The expander 21 is controlled to change the position of the laser spot in the Z direction. This allows to perform the crystalline lens surgery and the cornea surgery using one apparatus without exchanging the objective lens 24, the applicator 25, and a similar member to other members (such as a lens with a different focal length). In an optical layout of the expander 21, constituting the first lens group LE1 with the concave lens 81 and the convex lens 82 eliminates an intermediate image position in the expander 21. This reduces the length (length along the optical axis L) of the expander 21. This avoids increase in size of the apparatus 100.

While the operator selects the spot size of the laser spot from predetermined spot sizes in the above description, the configuration is not limited to this. Any other configuration is possible insofar as the operator sets (changes) the spot size. The operator may set the spot size (inputs a numerical value) through input means or similar means.

Next, a second embodiment of the present disclosure will be described. While the operator determines the spot size of the laser spot and the spot size is constant through the surgery in the aforementioned embodiment, the controller 70 sets the spot size based on depth information of the patient's eye E in the second embodiment. The controller 70 changes the spot size corresponding to an area of the laser irradiation during the surgery. Here, the observing optical system includes an optical coherence tomography unit (hereinafter referred to as OCT unit) to take a tomographic image of the crystalline lens as a depth information input unit that inputs information of the crystalline lens in the depth direction. The controller 70 changes the spot size of the laser spot in a position (an area) in the Z direction based on the information (depth information) of the tomographic image of the crystalline lens.

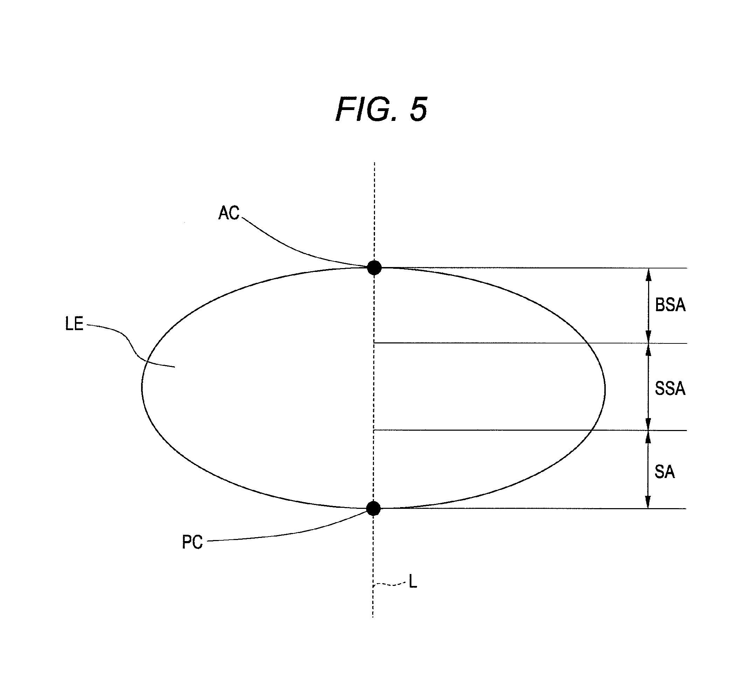

FIG. 5 is a schematic cross-sectional view illustrating a crystalline lens LE. The controller 70 acquires a tomographic image from the OCT unit, and then extracts a shape of the crystalline lens LE as illustrated in FIG. 5. The controller 70 detects a position of the anterior surface AC (at the front side) and a position of the posterior surface PC (at the back side) of the crystalline lens LE. The controller 70 determines (obtains) a laser irradiation area in the crystalline lens surgery that is divided into a safety area (first area) SA, a small spot area (second area) SSA, and a big spot area (third area) BSA in this order from the posterior surface PC toward the anterior surface AC between the anterior surface AC and the posterior surface PC.

The safety area SA is a laser non-irradiation area expecting an area where there is no damage on the posterior capsule by the laser irradiation. This area is assumed to be an area from the posterior capsule (posterior surface PC) to a predetermined position. The safety area SA is assumed to have the thickness of, for example, 100 µm to 500 µm.

The small spot area SSA is an area close to the posterior surface PC. In this area, the laser spot is set to a small spot size to reduce damage by the laser irradiation on the posterior capsule (posterior surface PC). Reducing the spot size of the laser spot ensures fine processing and shallow focal depth, thus reducing the damage on the posterior capsule. Additionally, the laser spot has large diameter of the laser beam that reaches the back side of the laser spot (back side of the eye). Even in the case where a breakdown at the laser spot has not been generated, this reduces a negative effect (damage due to high energy) on a retina. The small spot area SSA is assumed to be, for example, 100 to 10000 µm. The small spot area SSA is preferred to have set spot size that is relatively small, for example, less than 10 µm. Additionally, this spot size is preferred to be the minimum spot size (here, 5 µm) that can be set in the apparatus 100.

The big spot area BSA is an area close to the anterior surface AC (anterior capsule). The big spot area BSA is located relatively far from the posterior surface PC. Accordingly, even in the case where the focal depth of the laser spot is deep, the damage on the posterior surface PC is small. The big spot area BSA is located relatively far from the back side of the eye. Accordingly, even in the case where the focal depth of the laser spot is deep, the negative effect on the retina is small. The deeper focal depth of the laser spot allows to efficiently break up the crystalline lens LE (one shot allows to break up a region corresponding to the focal depth). In view of this, the spot size in the big spot area BSA is set as the thickness of the crystalline lens LE excluding the small spot area SSA and the safety area SA. In the big spot area BSA, it is preferred that large spot size, for example, equal to or larger than 10 µm, be set. Additionally, this spot size is preferred to be the maximum spot size (here, 15 µm) in the apparatus 100.

Accordingly, the controller 70 determines the spot size of the laser spot corresponding to the depth position of the crystalline lens LE. The controller 70 serves as spot size determining means and laser irradiation area setting means. When the laser irradiation is started, the controller 70 controls the expander 21 corresponding to the position of the laser spot in the Z direction. The controller 70 also controls the zoom units 84, 85, and 86 such that the spot size is formed corresponding to the preliminarily set area. In a sequence of surgery, the safety area SA is not irradiated with the laser. The small spot area SSA is irradiated using a laser spot that reduces damage on the posterior capsule with a small spot. The big spot area BSA is irradiated using a laser spot that efficiently breaks up the crystalline lens with a large spot size.

As described above, the spot size of the laser spot is changed in real time during a surgery. This ensures an efficient surgery.

Regardless of whether the spot size of the laser spot is set by the operator or the controller 70, the following configuration is possible. The controller 70 performs a following control based on the signal for inputting a change in the spot size and a value of preliminarily set spot size (for example, 10 µm) stored in the memory 71 in the case where the spot size is equal to or less than the predetermined spot size. This control is performed to reduce output power of the laser emitted from the laser unit 10 and increase repetition frequency.

A configuration that determines the spot size corresponding to a surgery mode may be possible. The controller 70 sets the spot size based on information that associates a surgery with spot size and is preliminarily stored in the memory 71, and a signal to set a surgery mode. Specifically, in the case where the surgery mode is the cornea surgery mode, the spot size is set as relatively small spot size (for example, 5 µm). On the other hand, in the case where the surgery mode is the crystalline lens surgery mode, the spot size is set as relatively large spot size (for example, 15 µm). As described above, a configuration that changes the spot size during the surgery in the crystalline lens surgery mode is possible. The depth information input unit is not limited to the OCT unit insofar as the depth information input unit acquires the tomographic image of the crystalline lens and information of the depth direction. For example, the depth information input unit may be Scheimpflug camera unit, an ophthalmic ultrasonic imaging unit, or a similar unit. The depth information input unit is not limited to the configuration embedded in the observing optical system 30. The depth information input unit may be a data input unit coupled to the apparatus 100 or have a configuration that acquires (receives) information of the depth direction of the crystalline lens from the measurement unit.

While the zoom units 84 and 85 change the focal length of the first lens group LE1 by movement of the lenses 81 and 82 along the optical axis in the above description, the configuration is not limited to this. Any other configuration is possible insofar as the configuration changes the focal length of the first lens group (unit) or discretely changes the focal length. For example, inserting and removing the lens on the optical axis of the first lens group may change the focal length. In this case, the focal length changing means includes a lens to be inserted and removed and a drive unit that drives the lens.

While the expander 21 includes three lenses in the above description, the configuration is not limited to this. Any other configuration is possible insofar as the configuration changes the beam diameter of the beam emitted from the expander 21 and also changes the incidence angle (NA). In this configuration, any optical element may be used. Specifically, any configuration is possible insofar as the focal length of at least one lens group of the first lens group and the second lens group is changed. For example, this configuration may change both the focal lengths of the first lens group and the second lens group. In this configuration, the first lens group may include two or more lenses, and the second lens group may include two or more lenses. While the first lens group (unit) is arranged at the upper stream side and the second lens group (unit) is arranged at the downstream side in the above description, the arrangement is not limited to this. The first lens group and the second lens group may have an opposite positional relationship. Additionally, the lens group may change the focal length using one lens. For example, fluid is filled between the anterior surface and the posterior surface of the lens. Changing the volume of the fluid may change the focal length of the lens. In this case, a unit that changes the volume of the fluid serves as the focal length changing means. Changing the voltage applied to liquid crystal may change the focal length of the lens. In this case, a unit that drives the liquid crystal using voltage serves as the focal length changing means.

While both of the laser output power and the repetition frequency are changed so as to change the irradiation condition of the laser unit 10 according to the spot size in the above description, the configuration is not limited to this. Any other configuration is possible insofar as the configuration reduces, for example, the distance between the spots due to the change in the spot size, and changes one of the aforementioned two conditions. This configuration does not necessarily change such irradiation conditions. In addition to changing the laser output power and the repetition frequency, the configuration may additionally change driving speed (scan rate) of the optical scanner 22. For example, changing the scan rate of the galvanometer mirror changes the scan rate of the laser spot. In the case where the spot size of the laser spot is small, changing the scan rate of the optical scanner 22 reduces it. This reduces the distance (spacing) between the laser spots, thus providing similar effects to those of the aforementioned configuration. In this case, the controller 70 controls the driving speed of the optical scanner 22 based on the setting of the spot size. At least one of the laser output power, the repetition frequency of the laser, and the driving speed of the optical scanner 22 may be simply changed based on the setting of the spot size. All the conditions do not necessarily need to be changed.

While the apparatus 100 may be used for performing the crystalline lens surgery and the cornea surgery in the above description, the configuration is not limited to this. Any other configuration is possible insofar as the configuration changes the position of the laser spot in the Z direction and the spot size of the laser spot. Any other configuration is possible insofar as the configuration performs one of the surgeries. The configuration does not necessarily perform the crystalline lens surgery and the cornea surgery, and any other configuration is possible insofar as the configuration makes an incision in an eyeball tissue and breaks up the eyeball tissue.

While the femtosecond pulse laser is used in the above description, the laser is not limited to this. Any other laser may be used insofar as the laser emits an ultrashort pulse laser beam such as a picosecond pulse laser that has the following characteristics. The characteristics include allowing for micron-order fine processing and internal processing on a transparent object, regardless of a material and without heating. A laser source that emits a YAG laser may be used.

While an ophthalmic laser surgical apparatus using the pulse laser is used in the above description, the configuration is not limited to this. Any other configuration is possible insofar as the configuration changes the spot size of the laser spot and also changes the position of the laser spot in the Z direction to perform the laser irradiation, thus performing a surgery and treatment of the patient's eye (eye of the patient). For example, the aforementioned configuration may be applied to a device (light coagulating device) that performs treatment using a continuous-wave laser.

As described above, the present disclosure is not limited to the embodiments. Various modifications and variations are possible with ingenuity by those skilled in the art. These configurations are included within the scope of the disclosure.

The foregoing detailed description has been presented for the purposes of illustration and description. Many modifications and variations are possible in light of the above teaching. It is not intended to be exhaustive or to limit the subject matter described herein to the precise form disclosed. Although the subject matter has been described in language specific to structural features and/or methodological acts, it is to be understood that the subject matter defined in the appended claims is not necessarily limited to the specific features or acts described above. Rather, the specific features and acts described above are disclosed as example forms of implementing the claims appended hereto.

## Claims

1. An ophthalmic laser surgical apparatus (100) for at least one of cutting and breaking up a tissue of a patient's eye using a laser, the ophthalmic laser surgical apparatus (100) comprising:
a laser source (10) configured to emit a pulse laser, the pulse laser achieving a breakdown in a spot (S1, S2, S3, S4) where the pulse laser is condensed; and
an irradiation optical system (20) configured to irradiate a target position with a laser, the irradiation optical system (20) including a moving optical system (21, 22), the moving optical system (21, 22) three-dimensionally moving a spot of the laser, wherein
the irradiation optical system (20) includes an objective lens (24), the objective lens (24) forming the spot of the laser in the target position,
the moving optical system (21, 22) includes:
an optical scanner (22) disposed in an upper stream of the objective lens (24), the optical scanner (22) being configured to scan the spot (S1, S2, S3, S4) of the laser on an XY plane perpendicular to an optical axis (L); and
a beam expander unit (21) disposed in an upper stream of the optical scanner (22), the beam expander unit (21) being configured to change a beam diameter of the laser, and change one of divergence and convergence of the laser, and
the beam expander unit (21) includes:
a first lens unit (LE1: 81, 82) configured to change a focal length so as to change the spot size of the laser in the target position;
a second lens unit (LE2: 83) including at least one lens; and
a plane-to-plane distance changing unit (84, 85, 86) configured to change a distance between a principal plane of the first lens unit (LE1) and a principal plane of the second lens unit (LE2) so as to adjust one of the divergence and the convergence of the laser that has passed through the first lens unit (LE1).

2. The ophthalmic laser surgical apparatus (100) according to claim 1, wherein
the first lens unit (LE1) includes a zoom unit (84, 85) that changes positions of at least two lenses (81, 82), a distance between the at least two lenses (81, 82) being changeable so as to change a focal length (F1), and
the plane-to-plane distance changing unit (84, 85, 86) relatively changes the distance between the principal plane (P1) of the first lens unit (LE1) and the principal plane (P2) of the second lens unit (LE2) along the optical axis (L).

3. The ophthalmic laser surgical apparatus (100) according to claim 2, wherein
the first lens unit (LE1) has a negative refractive power, the first lens unit (LE1) being located in an upper stream of the second lens unit (LE2), and
the second lens unit (LE2) has a positive refractive power.

4. The ophthalmic laser surgical apparatus (100) according to claim 3, wherein
the second lens unit (LE2) is fixedly disposed, and
the plane-to-plane distance changing unit (84, 85, 86) moves the first lens unit (LE1) along the optical axis (L).

5. The ophthalmic laser surgical apparatus (100) according to any one of claims 1 to 4, further comprising:
a controller (70) configured to control the plane-to-plane distance changing unit (84, 85, 86) based on a preliminarily set irradiation pattern, so as to move a position of the laser spot (S3, S4) along a Z direction perpendicular to the XY plane.

6. The ophthalmic laser surgical apparatus (100) according to claim 5, further comprising:
a focal length detector (84, 85) configured to detect the focal length (F1) of the first lens unit (LE1), wherein
the controller (70) is configured to change at least one of output power of the laser, repetition frequency of the laser, and driving speed of the optical scanner (22) according to the spot size of the laser spot (S1, S2) to be changed by the first lens unit (LE1), based on a detection result of the focal length detector (84, 85).

7. The ophthalmic laser surgical apparatus (100) according to claim 5 or 6, further comprising:
a surgery mode setting portion (63) configured to set:
a cornea surgery mode allowing the laser to make an incision in a cornea tissue; and
a crystalline lens surgery mode allowing the laser to make an incision in a crystalline lens tissue and break up the crystalline lens tissue, wherein
the controller (70) is configured to control the first lens unit (LE1) so as to set the laser spot (S1, S2) to a predetermined small spot size in a case where the surgery mode setting portion (63) sets the cornea surgery mode, and
the controller (70) is configured to control the first lens unit (LE1) so as to set the laser spot (S1, S2) to a spot size larger than the predetermined small spot size in a case where the surgery mode setting portion (63) sets the crystalline lens surgery mode.

8. The ophthalmic laser surgical apparatus (100) according to any one of claims 5 to 7, further comprising:
a depth information input unit (30) configured to input information of a depth direction of a crystalline lens (LE) of the patient's eye; and
a spot size determiner (70) configured to determine the spot size of the laser spot (S1, S2, S3, S4) corresponding to a depth position, based on the information of the depth direction from the depth information input unit (30), wherein
the controller controls the first lens unit (LE1) based on the spot size corresponding to the depth position so as to change the spot size of the laser spot (S1, S2, S3, S4), the depth position being determined by the spot size the determiner (70), and
the controller controls the plane-to-plane distance changing unit (84, 85, 86) to move a position of the laser spot (S1, S2, S3, S4) in the Z direction, and irradiate the crystalline lens (LE) with the laser.

9. The ophthalmic laser surgical apparatus (100) according to claim 8, further comprising:
a laser irradiation area setting portion (70) configured to determine:
a predetermined first area (SA) where a laser is not emitted from a posterior capsule (PC) of the crystalline lens toward an anterior capsule (AC) based on the information of the depth direction;
a second area (SSA) where the laser spot (S1, S2, S3, S4) has a predetermined small size at the anterior capsule (AC) side with respect to the first area (SA); and
a third area (BSA) where the laser spot (S1, S2, S3, S4) has a predetermined large spot size at the anterior capsule (AC) side with respect to the second area (SSA).

10. The ophthalmic laser surgical apparatus (100) according to claim 8 or 9, wherein
the depth information input unit (30) includes an optical coherence tomography unit, the optical coherence tomography unit being configured to take a tomographic image including the crystalline lens (LE).
